# EUROPEAN PATENT APPLICATION

(11) **EP 4 850 613 A1**
(43) Date of publication of application: **09.12.2026**
(21) Application number: 25773933.4
(22) Date of filing: 17.02.2025
(51) Int. Cl.: A61B 10/02

(54) **TISSUE SAMPLING NEEDLE**

(30) Priority: 18.03.2024 JP 2024042524
(71) Applicant: Terumo Kabushiki Kaisha, Tokyo 151-0072 (JP)
(72) Inventor: FUKUDA, Tomohiro, Ashigarakami-gun, Kanagawa 259-0151 (JP); KAMBARA, Kayo, Ashigarakami-gun, Kanagawa 259-0151 (JP); FUJIKI, Jo, Ashigarakami-gun, Kanagawa 259-0151 (JP)
(74) Representative: KIPA AB
(86) International application number: PCT/JP2025/005119
(87) International publication number: WO 2025/197379

(57) **Abstract**

Provided is a tissue sampling needle capable of preventing a load from being applied to cells of a biological tissue in a tapered portion.

A tissue sampling needle 100 includes a needle main body 110 in which a lumen 115 is formed, the needle main body includes a distal portion 120 having a needle tip 121 insertable into a biological tissue at a distal end and extending with a substantially constant inner diameter di1 in an axial direction, a proximal portion 130 disposed on a proximal end side of the distal portion and extending with a substantially constant inner diameter di2 in the axial direction, and a tapered portion 140 located between the distal portion and the proximal portion and having an inner diameter di3 increasing from a distal end side toward a proximal end side, in which an inner diameter of the distal portion is 0.5 mm or larger, and an average taper angle of the tapered portion is 5° or smaller.

## Description

### Technical Field

The present invention relates to a tissue sampling needle.

### Background Art

Conventionally, a procedure of sampling a biological tissue and various cells contained in the biological tissue using a tissue sampling needle has been performed.

Examples of the above-described procedure include a procedure of collecting an egg from the ovary transvaginally in infertility treatment and a procedure of diagnosing a lesion by observing cells of the lesion transdermally aspirated.

The tissue sampling needle desirably has a further reduced outer diameter in order to reduce pain of a patient associated with puncture. However, if an inner diameter (lumen diameter) also reduces as the outer diameter of the tissue sampling needle reduces, an excessive load is applied to the cells when the cells of the sampled biological tissue move in the lumen. In contrast, if a thickness of the tissue sampling needle is reduced in order to maintain the inner diameter while reducing the outer diameter of the tissue sampling needle, bending strength of the tissue sampling needle is reduced, so that deflection or the like is likely to occur in a procedure using the tissue sampling needle, and operability is reduced.

In order to solve the above-described problems, for example, it is conceivable to use a tapered needle in which an outer diameter and an inner diameter of a distal portion are made small, an outer diameter and an inner diameter of a proximal portion are made larger than those of the distal portion, and a tapered portion in which an outer diameter and an inner diameter gradually increase from the distal end side toward the proximal end side is provided between the distal portion and the proximal portion, as in a tissue sampling needle disclosed in Patent Literature 1. By forming the tissue sampling needle using the tapered needle, it is possible to reduce pain felt by a patient by the distal portion with a reduced diameter, and to prevent occurrence of the deflection of the tissue sampling needle by the proximal portion with an increased diameter.

### Citation List

### Patent Literature

Patent Literature 1: JP 2010-534332 A

### SUMMARY

### Technical Problem

However, if a taper angle of a tapered portion is large and an inner diameter of a tissue sampling needle changes steeply in the tapered portion, the following problem may occur.

When the taper angle of the tapered portion located between a distal portion and a proximal portion of the tissue sampling needle is extremely large and a portion such as a step is formed in a lumen, a flow such as a turbulent flow occurs in a flow of a biological tissue from the distal portion toward the proximal portion. As a result, an excessive load is applied to cells of the biological tissue.

The present invention is for solving the above-described problems, and an object thereof is to provide a tissue sampling needle capable of preventing application of a load to cells in a tapered portion.

### Solution to Problem

The above-described object of the present invention is achieved by the following means.

(1) A tissue sampling needle including:
   a needle main body in which a lumen is formed, the needle main body including:
   a distal portion having a needle tip insertable into a biological tissue at a distal end and extending with a substantially constant inner diameter in an axial direction;
   a proximal portion disposed on a proximal end side of the distal portion and extending with a substantially constant inner diameter in the axial direction; and
   a tapered portion located between the distal portion and the proximal portion and having an inner diameter increasing from a distal end side toward a proximal end side, in which
   an inner diameter of the distal portion is 0.5 mm or larger, and
   an average taper angle of the tapered portion is 5° or smaller.
(2) The tissue sampling needle according to (1), in which the inner diameter of the proximal portion is 1.0 mm or larger.
(3) The tissue sampling needle according to (1) or (2), in which the distal portion extends substantially constantly in the axial direction with an outer diameter of 1.1 mm or smaller, and
   the proximal portion extends substantially constantly in the axial direction with an outer diameter of 1.2 mm or larger.
(4) The tissue sampling needle according to any one of (1) to (3), in which a length in the axial direction of the distal portion is 10 mm or longer,
   a length in the axial direction of the tapered portion is 5.7 mm or longer, and
   a length in the axial direction of the proximal portion is 10 mm or longer.
(5) The tissue sampling needle according to any one of (1) to (4), in which the tapered portion includes:
   a first tapered portion extending from a proximal end of the distal portion toward the proximal end side; and
   a second tapered portion extending from a proximal end of the first tapered portion toward the proximal end side, and
   an average taper angle of the first tapered portion is larger than an average taper angle of the second tapered portion.
(6) The tissue sampling needle according to any one of (1) to (5), in which the tapered portion extends linearly or in a curved manner from the distal end side toward the proximal end side.

### Advantageous Effects of Invention

According to the tissue sampling needle described above, since an inner diameter of a distal portion is 0.5 mm or larger and an average taper angle of a tapered portion is 5° or smaller, it is possible to prevent a load from being applied to cells of a biological tissue when the sampled biological tissue moves in the distal portion and moves in the tapered portion.

### Brief Description of Drawings

[Fig. 1] Fig. 1 is a diagram illustrating a tissue sampling needle according to an embodiment of the present invention.
[Fig. 2] Fig. 2 is a cross-sectional view in an axial direction of a needle main body.
[Fig. 3] Fig. 3 is a cross-sectional view of the needle main body taken along arrows 3-3 illustrated in Fig. 2.
[Fig. 4] Fig. 4 is a cross-sectional view of the needle main body taken along arrows 4-4 illustrated in Fig. 2.
[Fig. 5] Fig. 5 is a cross-sectional view of the needle main body taken along arrows 5-5 illustrated in Fig. 2.
[Fig. 6] Fig. 6 is a partial enlarged view of a needle main body of an example and a needle main body according to a comparative example.
[Fig. 7] Fig. 7 is a graph illustrating results (simulation results of pressure loss) of the example and comparative example.
[Fig. 8] Fig. 8 is a graph illustrating results (simulation results of shear stress) of the example and comparative example.
[Fig. 9] Fig. 9 is an enlarged view illustrating a part of a needle main body according to Modification 1.
[Fig. 10] Fig. 10 is an enlarged view illustrating a part of a needle main body according to Modification 2.

### Description of Embodiments

An embodiment of the present invention will be described below with reference to the accompanying drawings. Note that the following description does not limit the technical scope or meaning of terms recited in claims. The dimensional ratios in the drawings are exaggerated for convenience of description and may be different from actual ratios.

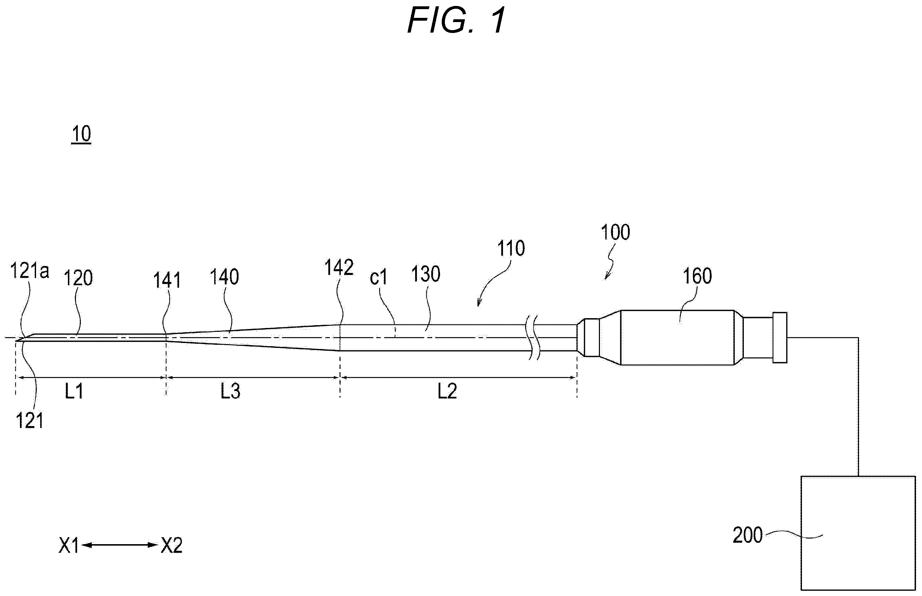

Hereinafter, a tissue sampling needle 100 according to an embodiment of the present invention will be described with reference to Figs. 1 to 5.

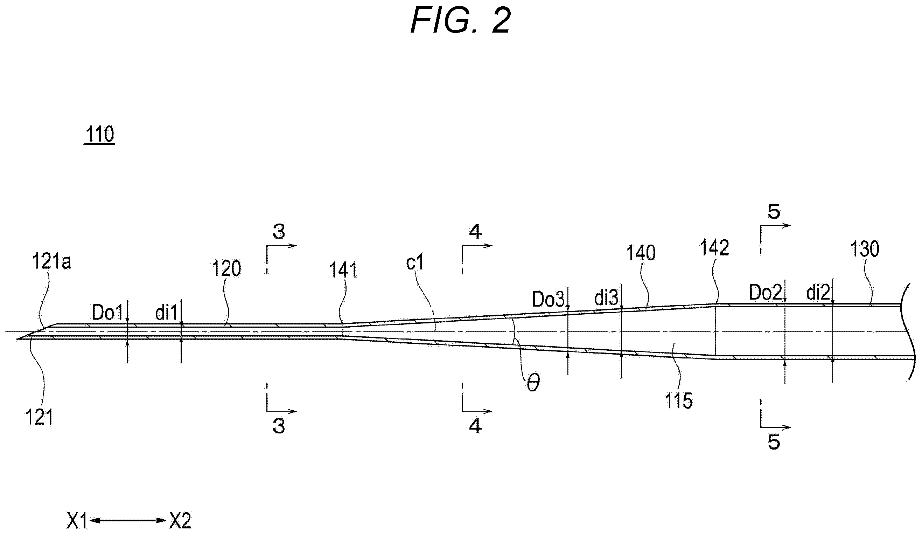

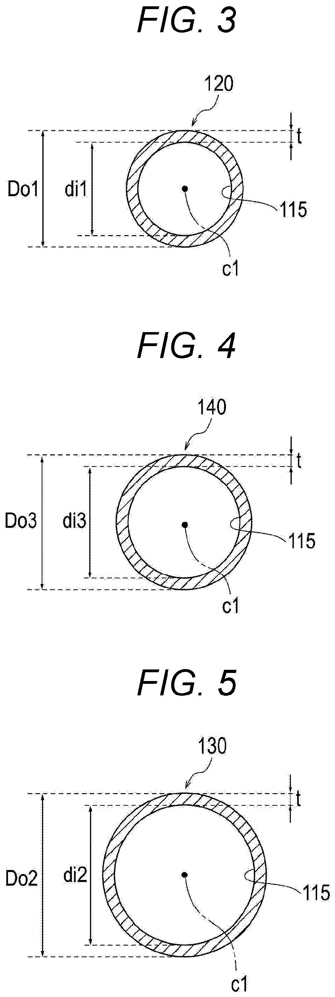

Fig. 1 is a diagram illustrating the tissue sampling needle 100 according to the embodiment and a tissue sampling device 10 including the tissue sampling needle 100 as a component. Fig. 2 is a partial cross-sectional view in an axial direction of a needle main body 110, and Figs. 3 to 5 are cross-sectional views (axially orthogonal cross-sectional views) of the needle main body 110 at respective portions indicated by arrows 3-3, 4-4, and 5-5 illustrated in Fig. 2.

In the description of the present specification, a direction in which the needle main body 110 extends is defined as an "axial direction", and is indicated by arrows X1 and X2 in each drawing. In the tissue sampling needle 100, a side that punctures a living body (a side indicated by arrow X1) is defined as a distal end side, and a side on which an operation is performed at hand (a side indicated by arrow X2) located on a side opposite to the distal end side is defined as a proximal end side.

### <Tissue Sampling Device 10>

As illustrated in Fig. 1, the tissue sampling device 10 includes the tissue sampling needle 100 and a suction unit 200.

The tissue sampling needle 100 includes the needle main body 110 that punctures a biological tissue. A lumen 115 (refer to Fig. 2) is formed inside the needle main body 110, and cells of the biological tissue can be sampled together with the biological tissue via the lumen 115. At a proximal portion 130 of the tissue sampling needle 100, a hub 160 that can be coupled to the suction unit 200 for generating a suction pressure (negative pressure) in the lumen 115 at the time of tissue sampling via a tube and the like is disposed.

The suction unit 200 used in the tissue sampling device 10 can be formed of, for example, a known pump mechanism used for sampling a biological tissue.

### <Tissue Sampling Needle 100>

As illustrated in Figs. 1 and 2, the tissue sampling needle 100 includes the needle main body 110 in which the lumen 115 is formed, and the hub 160 connected to a proximal end of the needle main body 110.

The tissue sampling needle 100 is formed as a medical instrument used to suck and sample cells via a distal end opening 121a formed at a needle tip 121 of the tissue sampling needle 100. When sampling the cells, the needle tip 121 of the needle main body 110 punctures a biological tissue of a predetermined biological organ. By operating the suction unit 200 in a state in which the needle tip 121 punctures the biological tissue, the cells of the biological tissue can be taken out of the living body via the lumen 115 of the tissue sampling needle 100.

The use of the tissue sampling needle 100 according to the present embodiment is not particularly limited, and this can be used for, for example, a procedure of sampling an egg from the ovary transvaginally in infertility treatment, or a procedure of diagnosing a lesion by observing cells of the lesion transdermally aspirated.

### <Needle Main Body 110>

As illustrated in Figs. 1 to 5, the needle main body 110 includes a distal portion 120 having the needle tip 121 capable of being inserted into the biological tissue formed at a distal end and extending with a substantially constant inner diameter di1 in the axial direction, the proximal portion 130 disposed on the proximal end side with respect to the distal portion 120 and extending with a substantially constant inner diameter di2 in the axial direction, and a tapered portion 140 located between the distal portion 120 and the proximal portion 130 and having an inner diameter di3 increasing from the distal end side toward the proximal end side.

The needle tip 121 can be formed as, for example, a known bevel needle. Note that, specific shape, structure and the like of the needle tip 121 are not particularly limited as long as the needle tip 121 can puncture the biological organ to be punctured.

The distal end opening 121a communicating with the lumen 115 is formed at the needle tip 121. The tissue sampling needle 100 can draw (suck) the biological tissue into the lumen 115 via the distal end opening 121a by operating the suction unit 200 in a state in which a certain range on the distal end side including the needle tip 121 is inserted into the biological organ.

The distal portion 120 linearly extends over a certain range on the proximal end side from the distal end at which the needle tip 121 is located. An outer diameter Do1 of the distal portion 120 can be made substantially constant in the axial direction.

The tapered portion 140 starts from a tapered portion distal end 141 located at the proximal end of the distal portion 120, and linearly extends so that the inner diameter di3 increases and reduces to a tapered portion proximal end 142 located at a distal end of the proximal portion 130. An outer diameter Do3 of the tapered portion 140 is configured to increase from the distal end side toward the proximal end side at an increasing rate substantially the same as that of the inner diameter di3 of the tapered portion 140.

The proximal portion 130 linearly extends over a certain range on the proximal end side from the tapered portion proximal end 142. An outer diameter Do1 of the proximal portion 130 can be formed to be substantially constant in the axial direction.

The lumen 115 is continuously connected along respective portions of the distal portion 120, the tapered portion 140, and the proximal portion 130. A central axis c1 of the needle main body 110 passes through the center (center of axially orthogonal cross section) of each of the portions 120, 130, and 140.

A material forming the needle main body 110 is not particularly limited, and for example, a metal material such as stainless steel, aluminum, an aluminum alloy, titanium, or a titanium alloy, or a resin material such as polyethylene, polypropylene, polymethyl methacrylate, polycarbonate, polyamide, or polyethylene terephthalate can be used.

A dimension example of each portion of the needle main body 110 will be described below.

The inner diameter di1 of the distal portion 120 can be set to 0.5 mm or larger. An average taper angle of the tapered portion 140 can be set to 5° or smaller.

Since the needle main body 110 has the inner diameter di1 of the distal portion 120 of 0.5 mm or larger, it is possible to reduce a pressure loss of the distal portion 120 when the suction pressure is generated in the lumen 115 of the needle main body 110. Therefore, it is possible to reduce the suction pressure generated for moving the sampled cells to the proximal end side in the lumen 115. Therefore, it is possible to reduce a load applied to the cells when the biological tissue moves in the distal portion 120.

The inner diameter di1 of the distal portion 120 is preferably 0.6 mm or larger, and more preferably 0.7 mm or larger from the viewpoint of reducing the pressure loss as described above.

Since the needle main body 110 is formed in such a manner that the average taper angle of the tapered portion 140 is 5° or smaller, the inner diameter di3 gradually increases gently between the tapered portion distal end 141 and the tapered portion proximal end 142 of the tapered portion 140. Therefore, the needle main body 110 can suppress a flow of a fluid flowing through the tapered portion 140 (the fluid containing the biological tissue generated by the suction of the suction unit 200) from becoming a turbulent flow greatly disturbed in the tapered portion 140. Therefore, the needle main body 110 can reduce the load applied to the cells when the sampled cells flow through the tapered portion 140.

The average taper angle of the tapered portion 140 is preferably 4° or smaller, and more preferably 3° or smaller from the viewpoint of suppressing the generation of the turbulent flow as described above.

The "average taper angle" in the present embodiment can be defined by twice the angle (refer to θ in Fig. 2) formed by the central axis c1 and a straight line representing a change rate of an inner radius obtained by (inner diameter [mm] of tapered portion proximal end 142 - inner diameter [mm] of tapered portion distal end 141)/2/length L3 [mm] in axial direction of tapered portion 140 (straight line length in the axial direction between the tapered portion distal end 141 and the tapered portion proximal end 142).

The inner diameter di2 (refer to Fig. 4) of the proximal portion 130 can be set to 1.0 mm or larger. Since the inner diameter di2 of the proximal portion 130 is set to 1.0 mm or larger, the pressure loss of the proximal portion 130 can be reduced when the suction pressure is generated in the lumen 115 of the needle main body 110.

The inner diameter di2 of the proximal portion 130 is preferably 1.1 mm or larger, and more preferably 1.2 mm or larger from the viewpoint of reducing the pressure loss as described above.

The distal portion 120 can be configured to extend substantially constantly in the axial direction with the outer diameter Do1 of 1.1 mm or smaller. The proximal portion 130 can be configured to extend substantially constantly in the axial direction with the outer diameter Do2 of 1.2 mm or larger.

Since the distal portion 120 of the needle main body 110 has the outer diameter Do1 of 1.1 mm or smaller, the outer diameter is reduced as compared with that of the conventional tissue sampling needle. Therefore, it is possible to reduce pain felt by a patient in a procedure using the tissue sampling needle 100. Since the proximal portion 130 of the needle main body 110 has the outer diameter Do2 of 1.2 mm or larger, the rigidity of the proximal portion 130 can be enhanced. Therefore, in the procedure using the tissue sampling needle 100, it is possible to prevent the needle main body 110 from being deflected, and to prevent deterioration in usability.

The outer diameter Do1 of the distal portion 120 is preferably 1.0 mm or smaller, and more preferably 0.9 mm or smaller from the viewpoint of reducing the pain felt by the patient as described above.

The outer diameter Do2 of the proximal portion 130 is preferably 1.3 mm or larger, and more preferably 1.4 mm or larger from the viewpoint of preventing occurrence of deflection during the procedure as described above.

The outer diameter Do3 of the tapered portion 140 can be any diameter according to a difference between the outer diameter Do1 of the distal portion 120 and the outer diameter Do2 of the proximal portion 130. Note that, in the tapered portion 140 of the present embodiment, the average taper angle of the inner diameter di3 may be set to 5° or smaller as described above, and for example, it is possible that the outer diameter Do3 does not have the average taper angle similar to that of the inner diameter di3.

A length L1 in the axial direction of the distal portion 120 can be set to 10 mm or longer. The length L3 in the axial direction of the tapered portion 140 can be set to 5.7 mm or longer. A length L2 in the axial direction of the proximal portion 130 can be set to 10 mm or longer. Note that, in the present embodiment, the length L2 in the axial direction of the proximal portion 130 is a linear distance from the tapered portion proximal end 142 to an exposed portion of the hub 160.

In a case where the portions of the needle main body 110 are formed to have the lengths L1, L2, and L3, respectively, as described above, the tissue sampling needle 100 can be formed to have an appropriate size that can be used as an egg collection needle. By setting the length L3 in the axial direction of the tapered portion 140 to 5.7 mm or longer, it is easy to set the average taper angle of the tapered portion 140 to a desired angle (5° or smaller), so that it is possible to provide the needle main body 110 that prevents the occurrence of the turbulent flow described above.

The length L1 in the axial direction of the distal portion 120 is preferably 20 mm or longer, and more preferably 30 mm or longer. The length L3 in the axial direction of the tapered portion 140 is preferably 7.1 mm or longer, and more preferably 9.5 mm or longer. The length L2 in the axial direction of the proximal portion 130 is preferably 100 mm or longer, and more preferably 130 mm or longer.

In a case where the lengths L1, L2, and L3 in the axial direction of the respective portions of the needle main body 110 are set as described above, the total length in the axial direction of the needle main body 110 is preferably 30 mm or longer and 400 mm or shorter, and more preferably 200 mm or longer and 350 mm or shorter.

A thickness t of the needle main body 110 can be made, for example, the same in the respective portions 120, 130, and 140 of the needle main body 110. The thickness t is not particularly limited, but can be set to, for example, 0.1 mm or longer from the viewpoint of securing the rigidity of the needle main body 110. The thickness t is preferably set to 0.12 mm or longer, and more preferably set to 0.14 mm or longer.

As described above, the tissue sampling needle 100 according to the present embodiment includes the needle main body 110 in which the lumen 115 is formed, the needle main body 110 includes a distal portion 120 having a needle tip 121 insertable into a biological tissue at a distal end and extending with a substantially constant inner diameter di1 in an axial direction, a proximal portion 130 disposed on a proximal end side of the distal portion 120 and extending with a substantially constant inner diameter di2 in the axial direction, and a tapered portion 140 located between the distal portion 120 and the proximal portion 130 and having an inner diameter di3 increasing from a distal end side toward a proximal end side, in which an inner diameter of the distal portion 120 is 0.5 mm or larger, and an average taper angle of the tapered portion 140 is 5° or smaller.

According to the tissue sampling needle 100 configured as described above, since the inner diameter of the distal portion 120 is 5 mm or larger and the average taper angle of the tapered portion 140 is 5° or smaller, it is possible to prevent a load from being applied to the cells of the biological tissue when the sampled biological tissue moves in the distal portion 120 and moves in the tapered portion 140.

### <Example>

Next, an example will be described. Note that the configuration of the present invention is not limited only to the contents of the example described below.

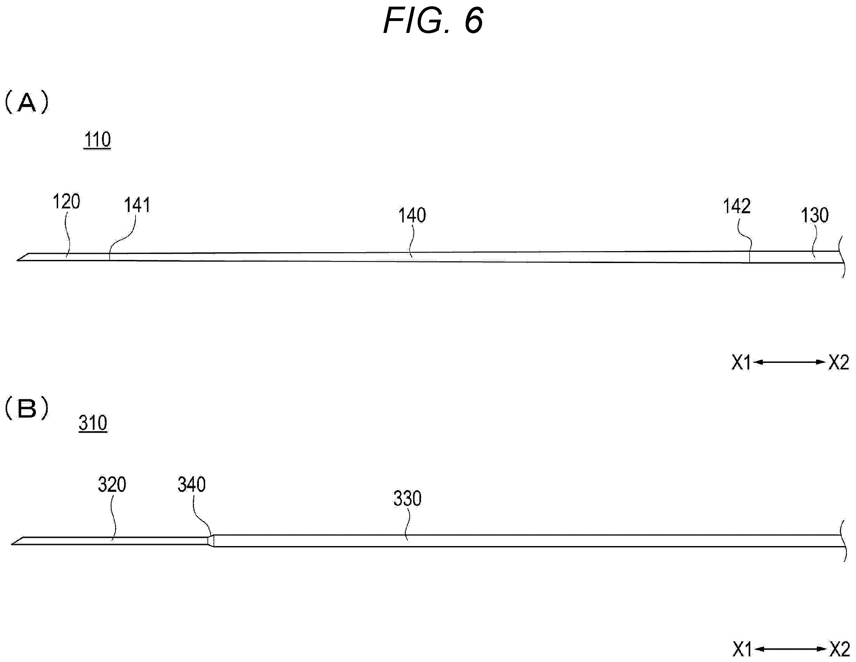

The following sample 1 was prepared as the needle main body 110 according to the example (refer to Fig. 6A).

### <Sample 1>

· Inner diameter di1 of distal portion 120 = 0.71 mm
· Inner diameter di2 of proximal portion 130 = 1.22 mm
· Average taper angle = 0.17°
· Length L1 in axial direction of distal portion 120 = 24 mm
· Length L3 in axial direction of tapered portion 140 = 170 mm

The following sample 2 was prepared as a needle main body 310 according to a comparative example (refer to Fig. 6B).

### <Sample 2>

· Inner diameter di1 of distal portion 320 = 0.67 mm
· Inner diameter di2 of proximal portion 330 = 1.20 mm
· Average taper angle = 14.84°
· Length L1 in axial direction of distal portion 120 = 50 mm
· Length L3 in axial direction of tapered portion 140 = 2 mm

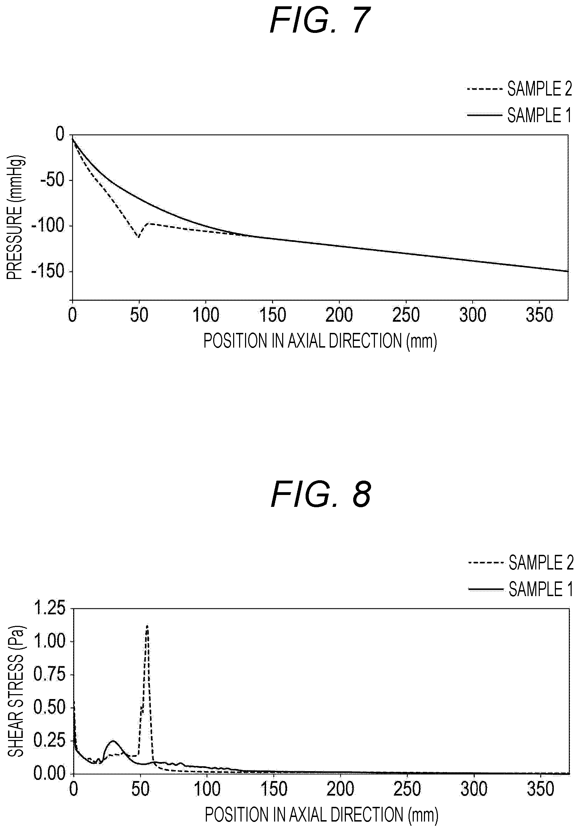

Fig. 7 illustrates results of simulation of an in-needle pressure performed under the condition of a volume flow rate of 50 mL/min for the sample 1 and sample 2.

In a graph in Fig. 7, the vertical axis represents the pressure, and the horizontal axis represents the position from the distal end of each of the needle main bodies 110 and 310. An inclination of the graph indicates a local pressure loss and an absolute value of the pressure at a position of 370 mm from the distal end indicates the pressure loss in an entire needle.

From the results illustrated in Fig. 7, since the inner diameter of the distal portion 120 of the sample 2 is smaller than that of the sample 1, the local pressure loss is large. In the vicinity of the tapered portion 340 (at a position of about 50 mm from the distal end), an extreme value of the pressure due to the disturbance caused by a rapid increase in the inner diameter of the needle main body 310 is observed.

In the sample 1, the inner diameter di1 of the distal portion 120 is 0.71 mm, which is larger than that of the distal portion 320 of the sample 2. Therefore, the local pressure loss at the distal portion is reduced. In the sample 1, the average taper angle of the tapered portion 140 is 0.17°, which is sufficiently smaller than that of the sample 2. Therefore, the extreme value of the pressure generated by the turbulence of the flow is not observed.

In order to reduce the pressure loss, it is necessary to lengthen a section having a large inner diameter in the entire needle. The sample 1 is disadvantageous in terms of pressure loss because the inner diameter gradually increases as compared with the sample 2. However, comparing the sample 1 and the sample 2, the pressure loss in the entire needle is comparable. This is considered to be because the inner diameter of the distal portion 120 of the sample 1 was formed to be larger than that of the sample 2.

Fig. 8 illustrates results of shear stress simulation performed on the sample 1 and sample 2.

In the graph in Fig. 8, the vertical axis represents the magnitude of the shear stress in the central axis calculated from a velocity distribution of a flow field formed in the lumen, and the horizontal axis represents the position from the distal end of each of the needle main bodies 110 and 310.

From the results illustrated in Fig. 8, it can be seen that the inner diameter of the needle main body 310 rapidly increases in the vicinity of the tapered portion 340 of the sample 2 (at a position of about 50 mm from the distal end), so that the shear stress in the flow field increases. In contrast, since the inner diameter of the needle main body 310 also increases in the vicinity of the tapered portion 140 of the sample 1 (position of about 24 mm from the distal end), the shear stress in the flow field tends to be slightly increased; however, since the average taper angle of the tapered portion 140 of the sample 1 is sufficiently smaller than that of the sample 2, it can be seen that the shear stress is greatly reduced as compared with that of the sample 2.

From the results of the example and comparative example, it has been confirmed that the needle main body 110 according to the sample 1 is formed in such a manner that the inner diameter of the distal portion 120 is larger than that of the needle main body 310 according to the sample 2 and the average taper angle is made smaller, so that the pressure loss can be suppressed to be comparable to that of the sample 2, and the shear stress applied to the biological tissue by the tapered portion 140 can be significantly reduced as compared with that of the sample 2.

Next, modifications of the above-described embodiment will be described. In the description of the modifications, detailed description of the contents already described in the above-described embodiment will be omitted.

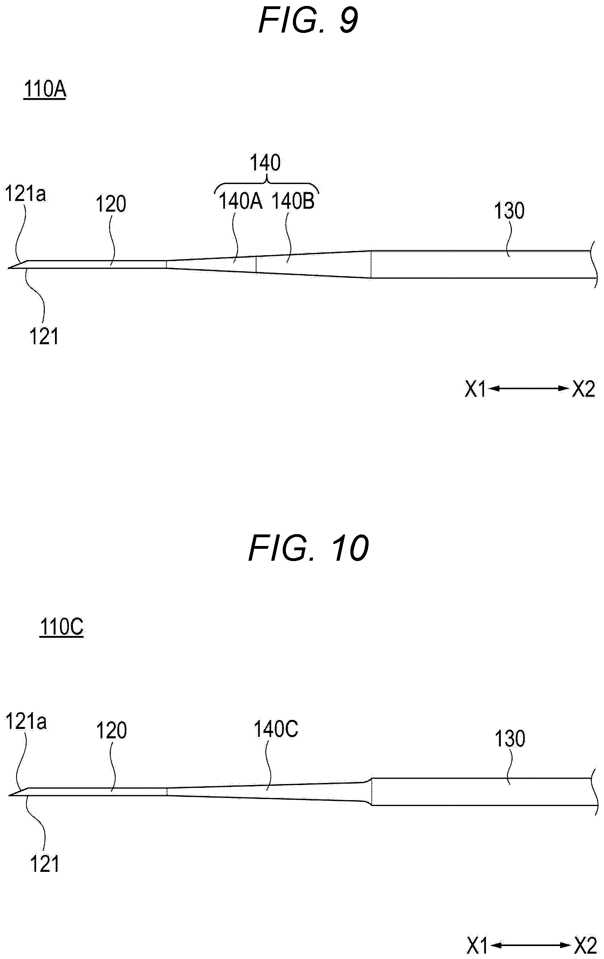

Fig. 9 illustrates a part of a needle main body 110A according to Modification 1.

As illustrated in Fig. 9, the tapered portion 140 may include a plurality of tapered portions. For example, as illustrated in Fig. 9, the tapered portion 140 may include two tapered portions 140A and 140B.

The tapered portion 140 according to Modification 1 includes a first tapered portion 140A extending from the proximal end of the distal portion 120 toward the proximal end side, and a second tapered portion 140B extending from a proximal end of the first tapered portion 140A toward the proximal end side.

The average taper angle of the first tapered portion 140A can be set to be larger than the average taper angle of the second tapered portion 140B. In the present modification, the average taper angle of each of the tapered portions 140A and 140B is 5° or smaller. The average taper angle of the first tapered portion 140A can be set to, for example, 5° or smaller, and the average taper angle of the second tapered portion 140B can be set to, for example, 4° or smaller.

As described in the present modification, the number of tapered portions provided in the needle main body 110A is not particularly limited as long as the average taper angle is 5° or smaller. For example, it is also possible to provide three or more tapered portions on the needle main body 110A.

Fig. 9 illustrates a part of a needle main body 110C according to Modification 2.

As illustrated in Fig. 9, a tapered portion 140C may extend in a curved manner from the distal end side toward the proximal end side, for example. The tapered portion 140C according to Modification 2 has a shape expanding so as to be separated from the central axis c1 from the distal end side toward the proximal end side. Even in a case of being configured as described above, it is possible to exert effects similar to those of the above-described embodiment as long as the average taper angle of the tapered portion 140C is 5° or smaller. As described above in the present modification, the shape of the tapered portion 140C (cross-sectional shape of the lumen 115 in the axial direction) is not limited only to the linear shape.

Although the tissue sampling needle according to the present invention has been described through embodiments and modifications, the present invention is not limited to each configuration described in the specification, and can be appropriately changed on the basis of the recitation in claims.

The present application is based on Japanese Patent Application No. 2024-042524 filed on March 18, 2024, the disclosure content of which is incorporated herein by reference in its entirety.

### Reference Signs List

- 10: Tissue sampling device
- 100: Tissue sampling needle
- 110: Needle main body
- 110A: Needle main body
- 110C: Needle main body
- 115: Lumen
- 120: Distal portion
- 121: Needle tip
- 121a: Distal end opening
- 130: Proximal portion
- 140: Tapered portion
- 140A: First tapered portion
- 140B: Second tapered portion
- 140C: Tapered portion
- 141: Tapered portion distal end
- 142: Tapered portion proximal end
- 160: Hub
- 200: Suction unit
- di1: Inner diameter of distal portion
- di2: Inner diameter of proximal portion
- di3: Inner diameter of tapered portion
- Do1: Outer diameter of distal portion
- Do2: Outer diameter of proximal portion
- Do3: Outer diameter of tapered portion
- c1: Central axis
- t: Thickness

## Claims

1. A tissue sampling needle comprising:
a needle main body in which a lumen is formed, the needle main body including:
a distal portion having a needle tip insertable into a biological tissue at a distal end and extending with a substantially constant inner diameter in an axial direction;
a proximal portion disposed on a proximal end side of the distal portion and extending with a substantially constant inner diameter in the axial direction; and
a tapered portion located between the distal portion and the proximal portion and having an inner diameter increasing from a distal end side toward a proximal end side, wherein
an inner diameter of the distal portion is 0.5 mm or larger, and
an average taper angle of the tapered portion is 5° or smaller.

2. The tissue sampling needle according to claim 1, wherein the inner diameter of the proximal portion is 1.0 mm or larger.

3. The tissue sampling needle according to claim 1, wherein the distal portion extends substantially constantly in the axial direction with an outer diameter of 1.1 mm or smaller, and
the proximal portion extends substantially constantly in the axial direction with an outer diameter of 1.2 mm or larger.

4. The tissue sampling needle according to claim 1, wherein a length in the axial direction of the distal portion is 10 mm or longer,
a length in the axial direction of the tapered portion is 5.7 mm or longer, and
a length in the axial direction of the proximal portion is 10 mm or longer.

5. The tissue sampling needle according to claim 1, wherein the tapered portion includes:
a first tapered portion extending from a proximal end of the distal portion toward the proximal end side; and
a second tapered portion extending from a proximal end of the first tapered portion toward the proximal end side, and
an average taper angle of the first tapered portion is larger than an average taper angle of the second tapered portion.

6. The tissue sampling needle according to claim 1, wherein the tapered portion extends linearly or in a curved manner from the distal end side toward the proximal end side.
